# EUROPEAN PATENT SPECIFICATION

(11) **EP 2 209 898 B1**
(45) Date of publication and mention of the grant of the patent: **04.01.2012**
(21) Application number: 08806243.5
(22) Date of filing: 12.09.2008
(51) Int. Cl.: C12N 15/90, A01K 67/027, C12N 9/02

(54) **TWO STEP CLUSTER DELETION AND HUMANISATION**
CLUSTER-DELETION UND HUMANISIERUNG IN ZWEI SCHRITTEN
DÉLÉTION EN DEUX ÉTAPES D'UN GROUPE DE GÈNES ET HUMANISATION

(30) Priority: 14.09.2007 GB 0718029
(43) Date of publication of application: 28.07.2010
(73) Proprietor: ITI Scotland Limited, Glasgow Strathclyde G2 5SG (GB)
(72) Inventor: SCHEER, Nico, 50999 Köln (DE)
(74) Representative: Goodfellow, Hugh Robin
(86) International application number: PCT/GB2008/003084
(87) International publication number: WO 2009/034328

(56) References cited:
- EP-A- 1 206 906
- WO-A-2005/019463
- WO-A-2006/064197
- WO-A-2008/081197
- WALLACE HELEN A C ET AL: "Manipulating the mouse genome to engineer precise functional syntenic replacements with human sequence" CELL, CELL PRESS, CAMBRIDGE, NA, US, vol. 128, no. 1, 11 January 2007 (2007-01-11), pages 197-209, XP002481713 ISSN: 0092-8674 cited in the application
- WOLF C ROLAND ET AL: "The application of transgenic and humanised nuclear receptor models in drug development" DRUG METABOLISM REVIEWS; 9TH EUROPEAN MEETING OF THE INTERNATIONAL-SOCIETY-FOR-THE-STUDY-OF-XEN OBIOTICS (ISSX), MARCEL DEKKER, NEW YORK, NY, US; MANCHESTER, UK, vol. 38, no. Suppl. 1, 1 January 2006 (2006-01-01), pages 7-8, XP008095774 ISSN: 0360-2532
- AL-HASANI KEITH ET AL: "Complementation of alpha-thalassaemia in alpha-globin knockout mice with a 191 kb transgene containing the human alpha-globin locus" TRANSGENIC RESEARCH, LONDON, GB, vol. 13, no. 3, 1 June 2004 (2004-06-01), pages 235-243, XP002481712 ISSN: 0962-8819
- BRAULT V, BESSON V, MAGNOL L, DUCHON A, HÉRAULT Y.: "Cre/loxP-mediated chromosome engineering of the mouse genome." HANDB EXP PHARMACOL., no. 178, 2007, pages 29-48, XP002516469
- ANDRÉ OUMARD ET AL: "Recommended Method for Chromosome Exploitation: RMCE-based Cassette-exchange Systems in Animal Cell Biotechnology" CYTOTECHNOLOGY, KLUWER ACADEMIC PUBLISHERS, DO, vol. 50, no. 1-3, 14 June 2006 (2006-06-14), pages 93-108, XP019393904 ISSN: 1573-0778
- VAN HERWAARDEN AE ET AL.: "Knockout of cytochrome P450 3A yields new mouse models for understanding xenobiotic metabolism" J CLIN INVEST., vol. 117, no. 11, 1 November 2007 (2007-11-01), pages 3583-3592, XP002515970

## Description

### Field of the invention

The present invention relates, in general, to a method for humanising a mouse. In particular, the invention relates to a method for replacing a cluster of mouse genes with single or multiple genes from the corresponding human cluster using a combination of homologous recombination and site-specific recombination.

### Background to the invention

Mouse models are an invaluable tool for investigating human disease, and are used extensively to study the progression of many diseases, and to test potential therapeutics. Conventionally, transgenic mice have been produced through pronuclear injection of exogenous DNA. More recently, mice have been generated by fusing an embryonic stem cell with a cell containing a Bacterial Artificial Chromosome (BAC) or a Yeast Artificial Chromosome (YAC) comprising the exogenous gene of interest and a selectable marker to assess integration of the exogenous DNA segment into the embryonic cell genome, as described in WO94/02602, for example. Such methods rely on integration of the BAC or YAC into the embryonic stem cell genome through the process of homologous recombination. Due to the technical demand in handling BACs and YACs, and the low transfection rates of ES cells by using large DNA constructs, transgenesis in this manner is time-consuming, inefficient and inaccurate.

Wallace et al. (Cell 128, 197-209 2007) recently described a method known as recombinase-mediated genomic replacement (RMGR). This method utilises site-specific recombination to replace a mouse allele with the human allele of the orthologous gene. Two non-interacting site-specific recombination sites (*loxP* and *lox511*) are inserted into the mouse chromosome flanking the target gene by homologous recombination. An identical pair of non-interacting site specific recombination sites are inserted into a BAC so as to flank the human allele of the target gene. The introduction of the BAC into the mouse cell, and the subsequent expression of the site-specific recombinase result in two site-specific recombination reactions between the compatible site-specific recombination sites of the BAC and the mouse chromosome (*loxP*/*loxP* and *lox511*/*lox511*), and the reciprocal exchange of the human gene sequence for the mouse sequence. However, this method contains multiple steps that must be performed in an embryonic stem cell, leading to inefficiencies, largely because the karyotype of the cell is often disrupted. There thus remains a need for a streamlined method for humanising a mouse, which would ideally require fewer steps to be performed in an embryonic stem cell, and thereby overcome some of these problems of inefficiency.

In addition, the invention sets out to resolve another problem in the field of humanised mouse generation. For instance, previously-described methods for generating humanised mouse models have focused merely on replacing a single endogenous mouse gene with the corresponding human gene. This is an acceptable method for those gene loci where one mouse gene corresponds to one human gene. However, in evolution, clusters of genes may develop, that often undergo repeated changes such as duplication and diversification, so that the cluster may vary significantly between different species. Therefore, within a cluster it is sometimes not possible to identify truly orthologous genes between species. In extreme cases, there may be only one functional gene(s) in a cluster of one species, but many more in another. One such example is the CYP2D gene cluster of metabolic enzymes, which exhibits only one functional gene in humans (CYP2D6), but nine functional genes in mice. In such cases, replacing a single mouse gene with the orthologous human gene by the conventional gene targeting mechanism of homologous recombination will not result in functional humanisation due to expression of residual genes from the mouse cluster.

Furthermore, in many cases, the gene product of one gene can constitute the major functionality of the whole cluster (e.g. CYP3A4 in the human CYP3A cluster or CYP2C9 in the human CYP2C cluster) and it might be impossible to identify a functional homologue of this gene in another species. This causes uncertainty as to which gene should be replaced, and by what. Additionally problematic is the reality that deletion of a large fragment of DNA from the mouse chromosome, which constitutes a whole gene cluster, may not be possible as a single event due to the inefficiency of homologous recombination if the targeting arms are too far apart on the mouse chromosome.

A method of generating humanised mouse models in which the humanised locus successfully mirrors the equivalent human gene loci of interest would allow for a more realistic investigation into the functions of genes encoded at such loci, and facilitate research into the therapeutic intervention of conditions that are associated with genes that form part of gene clusters of similar functionality.

It is known in the art that site-specific recombinases can be utilised to target homologous recombination to specific chromosomal locations (see Jessen et al., 1997). The use of such site specific recombinases allows recombination to be initiated upon demand by the addition of the site-specific recombinase.

US2007/0061900 describes a method for the humanisation of the heavy and light chain immunoglobulin variable region gene loci. This method involves the insertion into each of two vectors, termed LTVECs, of a site-specific recombination site arranged so as to be contiguous to a portion of the human immunoglobulin variable region. These LTVECs are then linearised and introduced into the genome of a mouse cell by homologous recombination, so that the site-specific recombination sites flank the mouse immunoglobulin variable region sequences, and the partial human immunoglobulin variable region sequences flank the site-specific recombination sites. Effecting site-specific recombination excises the mouse immunoglobulin variable region sequence and joins the two partial human immunoglobulin variable region sequences, with the residual site-specific recombination site contained within it. The resulting mice produce hybrid antibodies containing human variable and mouse constant regions, with subsequent transformation steps required to allow production of pure human antibodies. The segmental nature of the immunoglobulin variable region allows the residual site-specific recombination site to remain within the nucleic acid sequence with little chance of a detrimental effect. However, there is no indication that this technology might be utilised for the humanisation of non-segmental genes, where the presence of a nucleic acid sequence coding for a residual site-specific recombination site within the gene would eliminate its ability to be transcribed. The application WO2006064197 discloses a method of humanising a mouse for a gene cluster of interest comprising the steps of two consecutive targeting rounds in ES cells for incorporating a pair of site-specific recombination sites into the mouse genome such that the mouse target sequence that is to be replaced is flanked on each side by a recombination site with two kinds of targeting vectors. Cre-mediated deletion *in vivo* results in a knock out of the loxP-flanked murine gene cluster. A further step of Cre-mediation recombination with BAC-DNA comprising the human replacement gene was necessary to introduce human sequences by Cre-mediated insertion into re-derived ES cells. The selection cassettes flanking the humanized clusters can be removed by FLP mediated deletion. In contrast, the method of the present invention allows the complete humanisation of any mouse gene or gene cluster through just two rounds of targeting in embryonic stem cells.

### Summary of the invention

According to the present invention, there is provided a method of humanising a mouse for a gene of interest, comprising:
a) incorporating a pair of site-specific recombination sites into the mouse genome by homologous recombination such that the mouse target gene sequence that is to be replaced is flanked on each side by a recombination site;
   wherein at least one of the two recombination sites is constructed so as to be contiguous with a human replacement gene sequence;
   and said human replacement gene sequence is positioned so that said recombination site lies between said human replacement gene sequence and said mouse target gene sequence;
b) effecting recombination between the site-specific recombination sites such that the human sequence is introduced at the position in the mouse chromosome previously occupied by the mouse target gene, flanked by the residual site-specific recombination site.

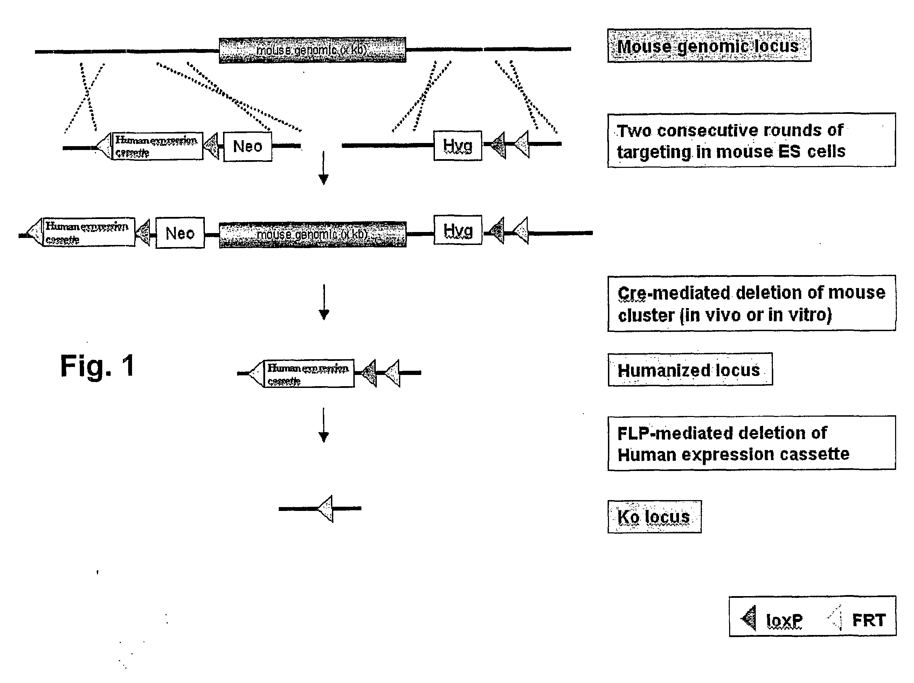

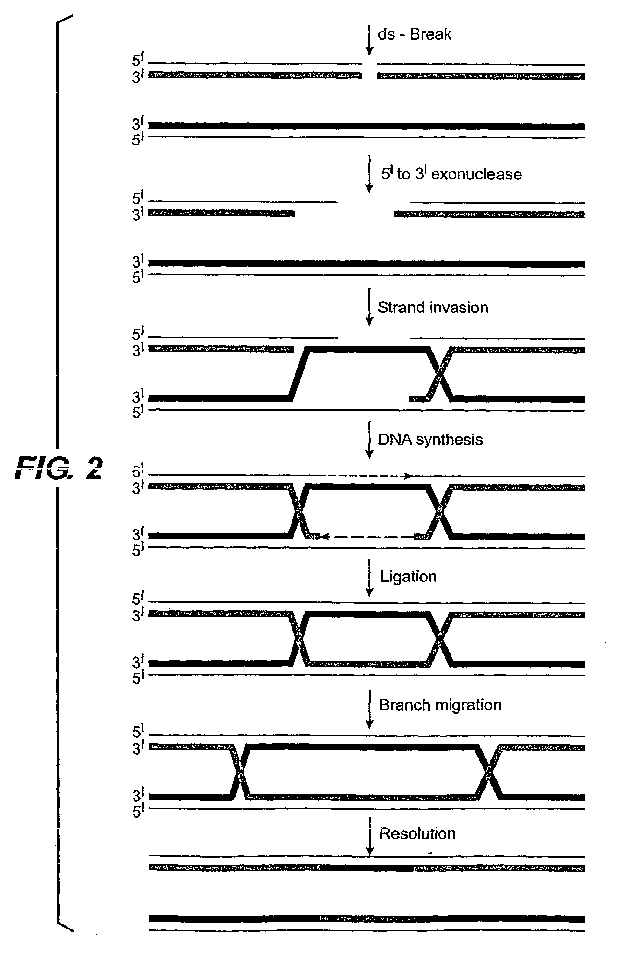

A simple schematic of the mechanism of the invention is shown in Figure 1. In brief, two site-specific recombination sites are incorporated into the mouse chromosome in embryonic stem cells by two separate conventional homologous recombination reactions. Homologous recombination is a phenomenon well known in the art, yet for assistance in understanding, a schematic of the mechanism of homologous recombination is shown in Figure 2. The two recombination reactions are facilitated by short regions of homology between the target mouse chromosome and the replacement nucleic acid sequence that comprises the site-specific recombination site. These regions of homology facilitate strand invasion and subsequent base pairing, allowing strand elongation which inserts each recombination site into the mouse chromosome.

At one side or both sides of the mouse target sequence, in addition to a site-specific recombination site, the replacement nucleic acid sequence is linked to a human gene sequence that is intended to replace the mouse target sequence. Therefore, the complete human replacement gene sequence and the attached site-specific recombination site are inserted concurrently into the mouse chromosome, resulting in the arrangement shown in Figure 1. Once both recombination reactions are complete, the site-specific recombination sites should thus flank the mouse target gene. Both recombination sites should be aligned in the same direction, as shown in Figure 1, so as to allow their recombination together in due course.

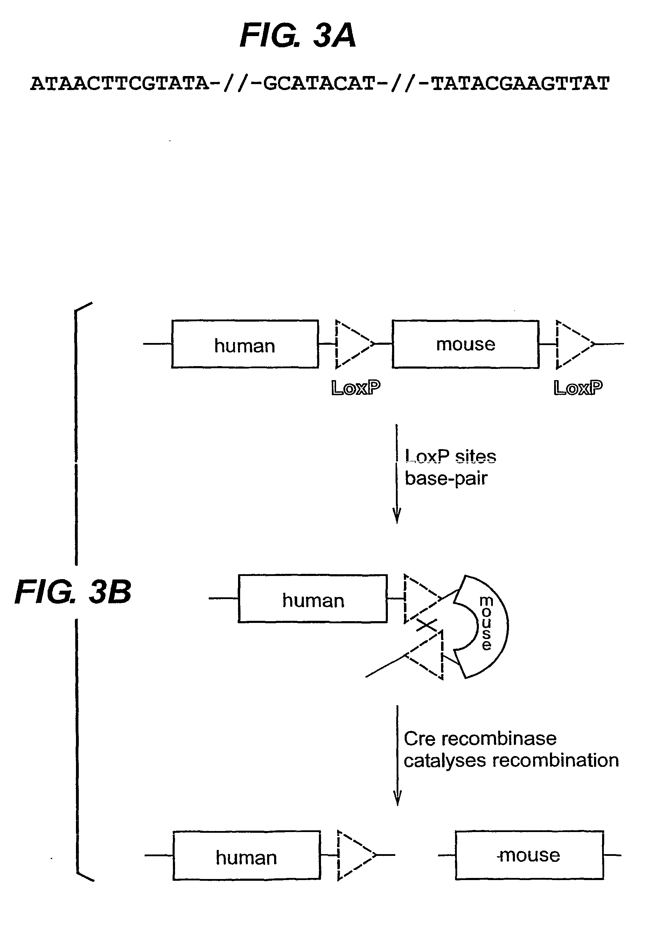

In order to excise the mouse target gene sequence and replace it with the replacement human gene sequence, site-specific recombination is then effected between the two recombination sites. The mechanism is depicted in Figure 3. These recombination events result in excision of the mouse target gene sequence, leaving the human replacement gene sequence positioned in the mouse chromosome that was previously occupied by the mouse target gene, flanked by the residual site-specific recombination site formed by the recombination event.

The method of the invention has a number of advantages. For one thing, it does not necessarily require handling with BACs or YACs and it significantly facilitates the generation of humanised mice, since the transformation steps that are required at the embryonic stem cell stage are reduced in number, even over the method of Wallace described above. In the method of the invention, a minimum of two rounds of conventional targeting through homologous recombination are required in embryonic stem cells, namely the incorporation of the site-specific recombination site at one side of the target sequence incorporation of the site-specific recombination site + human gene(s) on the other side. Then all that is required is a single deletion step, in which the mouse target sequence is removed. This can be done *in vitro* or *in vivo*. This methodology is more efficient in that it requires at least one step less than the Wallace method, which under usual practice translates into 8 or so weeks less work at the ES cell stage, and which significantly reduces the risk of accumulating disruptions in the ES cells during multiple rounds of transfection that could render these cells incapable of passing the germline.

There are also other advantages in terms of efficiency at later stages of the methodology, since humanisation of a gene or gene cluster by simultaneous replacement of the endogenous mouse gene or cluster according to the invention ensures that the human gene is situated at the same site as the mouse gene that it replaces. Accordingly, when crossing to homozygosity, there is only one locus that needs replacement. In contrast, in order to knock out a mouse gene and replace its function with a human gene situated on a different chromosome, there is a much more complex genetic background. Additional numbers of crosses will be required in order to achieve homozygosity, both of the mouse knock-out and the human replacement.

A particular advantage of the method of the invention is in terms of its efficiency. As is evidenced from the Wallace work, it is extremely inefficient to attempt to use recombinase-mediated recombination to replace a large segment of chromosomal DNA with a similarly-sized segment on a vector. Efficiencies are reported as of the order of 1 in 10⁸. In contrast, the intramolecular recombination event that is selected for in the course of the present invention is usually of the order of 1 in 10⁶. Furthermore, because the lengths of DNA used by Wallace are so long (of the order of 200kb), there is a much increased opportunity for intramolecular rearrangements and undesired homologous recombination events to occur, which increases the chance of a non-functional or incorrect DNA structure being created. These problems are avoided using the method of the present invention.

The method has the result of generating a mouse which expresses a human protein or proteins in the absence of the equivalent mouse gene or genes; these are deleted from the mouse chromosome during the course of the methodology.

The introduced human gene may be incorporated under the control of its own regulatory sequences. Alternatively, the genetic recombination events can be arranged so that the equivalent mouse regulatory sequences are situated upstream of the human sequence and thus used in their place. Sometimes it will be desired to retain the mouse regulatory sequences rather than incorporate the supposed human regulatory sequences with the gene coding sequence. For example, in the case of CYP3A4, a humanised mouse has been created which carries the human promoter and the activity of this governs regulation of the CYP3A4 gene. However, Cheung et al (Journal of Pharmacology and Experimental Therapeutics 316, 1328-1334 2006) demonstrate that under this arrangement, the CYP3A4 protein is not expressed in adult males. It is thus supposed that some promoter element or other factor must exist that is missing from the construct used. In contrast, by retaining the mouse regulatory sequences and using these for regulation instead of the human sequences, one can guarantee that faithful regulation of the introduced gene will be retained and such problems avoided.

One advantage of the methodology of the invention over conventional techniques, where integration of human genes into the mouse chromosome is more or less random, is that integration at the site of the equivalent mouse genes ensures that the genomic context of gene placement is retained. By integrating at such a site, it is likely that the local chromosome structure is "open" in the sense that access to the chromosomal DNA is possible for transcription factors and other proteins required for transcription to take place. Not only this, but the same chromosomal context is retained as for the endogenous mouse gene, such that regulation of DNA transcription at the level of the tertiary structure of the chromosome, by way of histone binding, and local folding/unfolding of the chromosome, is retained. This ensures that holistic regulation of gene transcription is retained, such that the same tissue distribution of gene regulation is followed for the introduced human gene as that seen for the endogenous mouse gene. This complete retention of physiological regulation mechanisms at the gene transcription level is not common to prior art techniques.

A further advantage of the methodology relates to the problem of lethality, generated by deletion of an important gene or gene cluster, especially if there is a haplo-insufficiency with respect to the deleted gene or gene cluster, which causes lethality in heterozygote animals. Because the methodology of the invention simultaneously deletes the mouse gene(s) and replaces this with its human functional equivalent, there is no time when the function encoded by the gene is compromised. Accordingly, provided that function of the human protein does indeed replace that of the equivalent mouse protein, problems of this nature are avoided.

The invention also allows the formation of a functional human gene by combining two component halves that are introduced either side of a mouse gene or cluster on the chromosome. Upon site-specific recombination to excise the mouse gene or cluster, the component halves of the human gene are brought together so as to form a functional human gene. The potential problem of the residual RT site disrupting coding sequence can be resolved by placement of this RT site within an intron. Equally, the invention allows the combination of different genes usually not positioned contiguously on a human chromosome. Of course, this strategy could not work using prior art techniques such as that of Wallace, where the components to be combined might be on different chromosomes or too far apart to be covered by a single BAC or YAC.

The approach described herein may be utilised particularly advantageously in situations where a particular mouse and human functionality is coded for by a number of different genes, linked contiguously in the chromosome in a cluster. The method allows the simple replacement of the mouse gene cluster for the equivalent human gene or cluster. Similarly, and for the same reasons, the method of the invention is well suited to humanisation of a mouse genetic system which contains a high level of redundancy at the genetic level. For instance, where the function of one mouse gene can be taken over in full or in part by another mouse gene in the same gene cluster in the absence of the first choice gene, it is important when humanising to ensure that all genes are replaced by the human equivalent(s); if this is not done, then the readout of the humanised system will be confused and ultimately meaningless.

### Brief description of the figures

**Figure 1****. Schematic of the methodology of the invention.** The method provides a mechanism for humanisation, comprising the insertion of two pairs of site-specific recombination sites, one of which is arranged so as to be contiguous to a human gene replacement sequence, through homologous recombination, and effecting site-specific recombination between the first pair of sites to excise the mouse target gene. Effecting recombination through the second pair of site-specific recombination sites excises the human gene replacement sequence, creating a gene knock-out.
**Figure 2****. Mechanism of homologous recombination.** Homologous recombination occurs following a double stranded chromosomal break. 5' to 3' exonuclease activity produces a 3' overhang and allows strand invasion to occur. DNA synthesis utilises the intact strand as a template and ligation repairs the chromosomal break generating a Holliday junction. Subsequent branch migration and resolution produce recombinant products.
**Figure 3****. A) The LoxP site-specific recombination site. B) Mechanism of site-specific recombination.** Two LoxP sites align through complementary base pairing, allowing Cre recombinase to catalyse recombination between the 2 sites, so excising the mouse target gene.

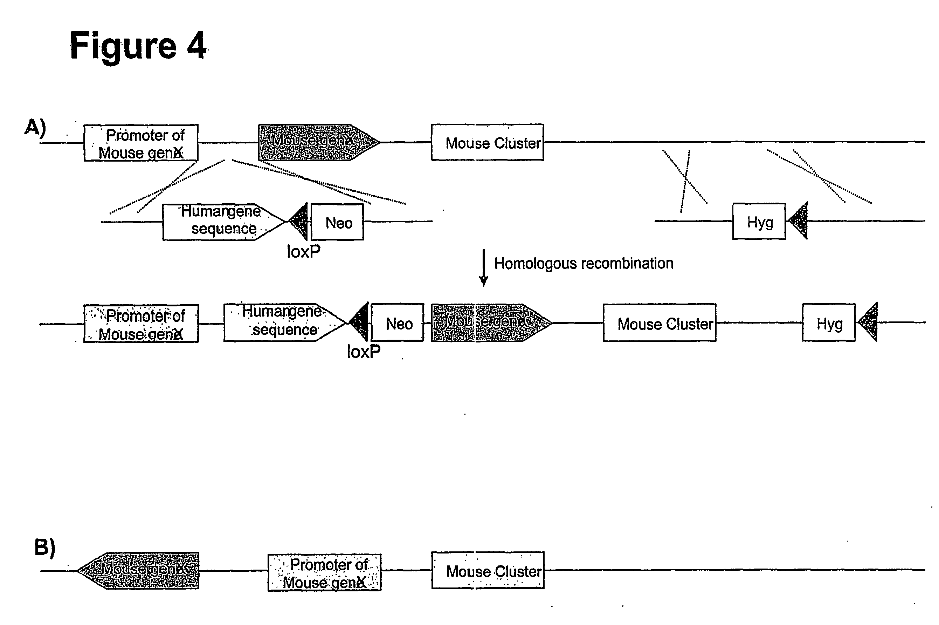

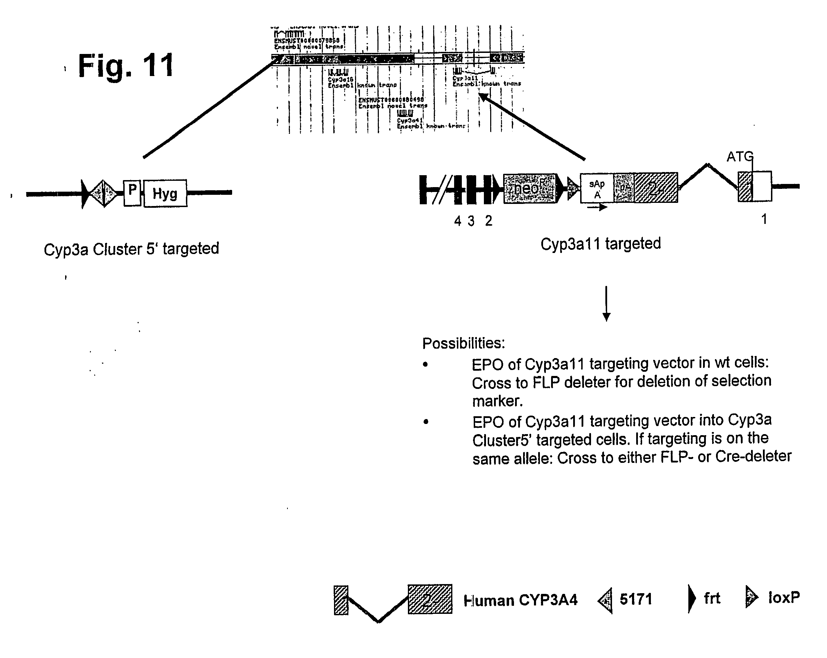

**Figure 4****. Principles of the invention if expression of the human gene is to be controlled by a mouse promoter wherein A) a suitable mouse promoter is oriented inwards at one end of the mouse cluster or B) a suitable mouse promoter is oriented outwards at one end of the mouse cluster. A)** The human replacement gene sequence is inserted between the mouse promoter and the mouse target gene, allowing the mouse promoter to persist, and to control expression from the human gene replacement sequence This strategy was chosen in case of CYP3A4 into CYP3A11 (see Figure 11). Additional FRT-sites have been included for cloning purposes only. **B)** The strategy is not applicable to the method of the invention. Therefore, and also because a suitable promoter is not necessarily located at the end of a mouse cluster, the use of human promoters, as depicted on the following figures, or even the re-introduction of mouse promoters can be applied much more universally.

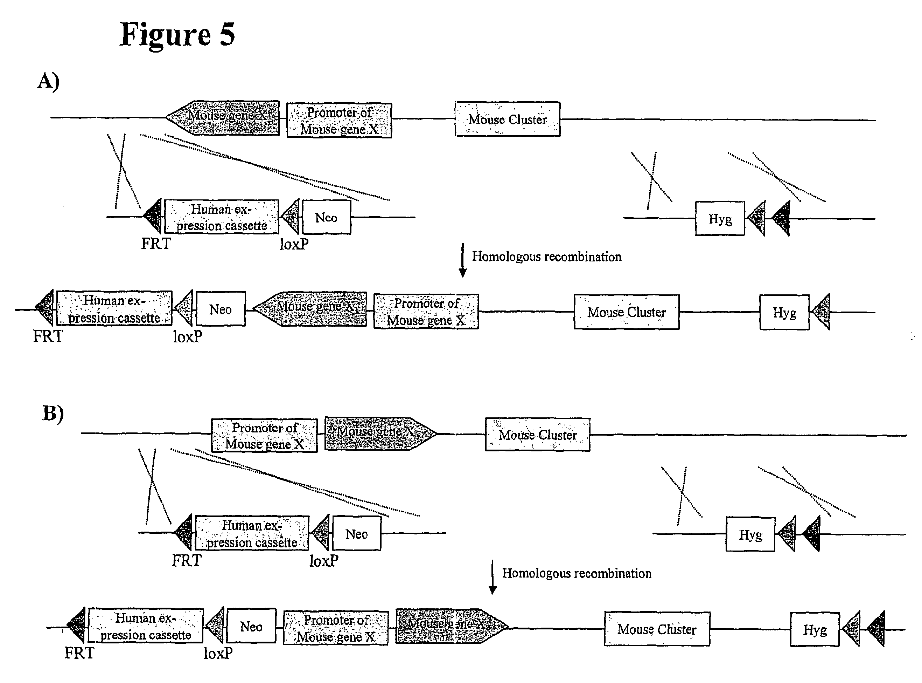

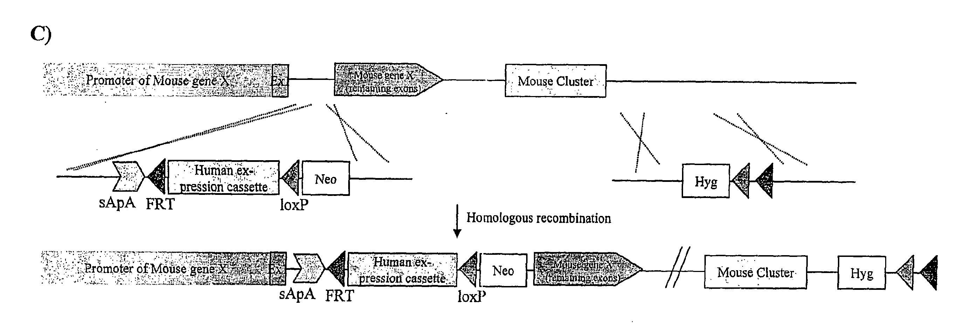

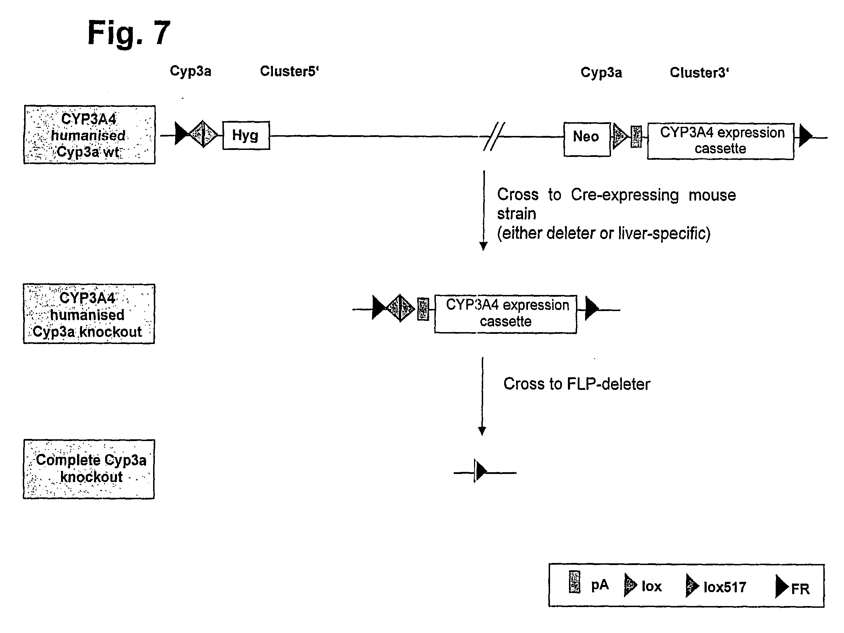

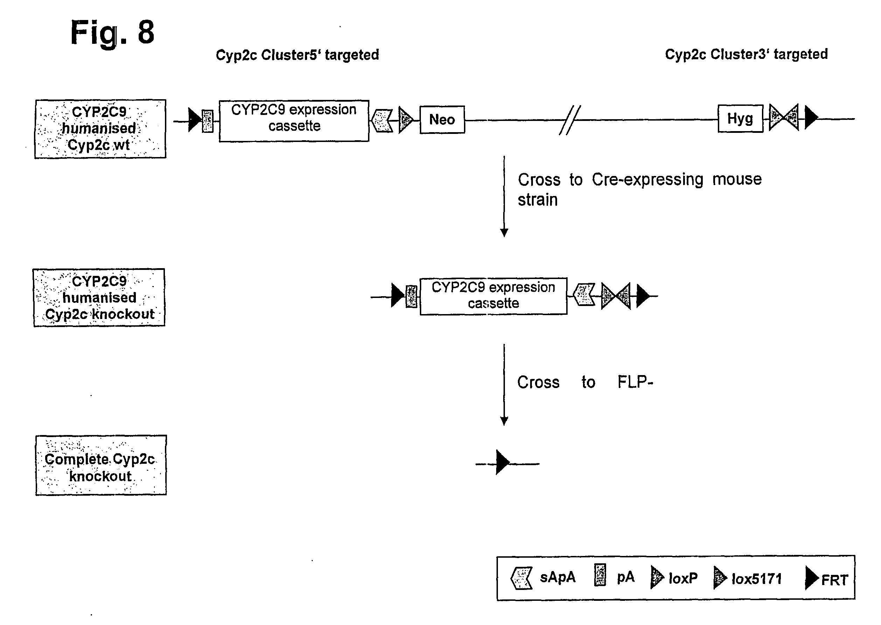

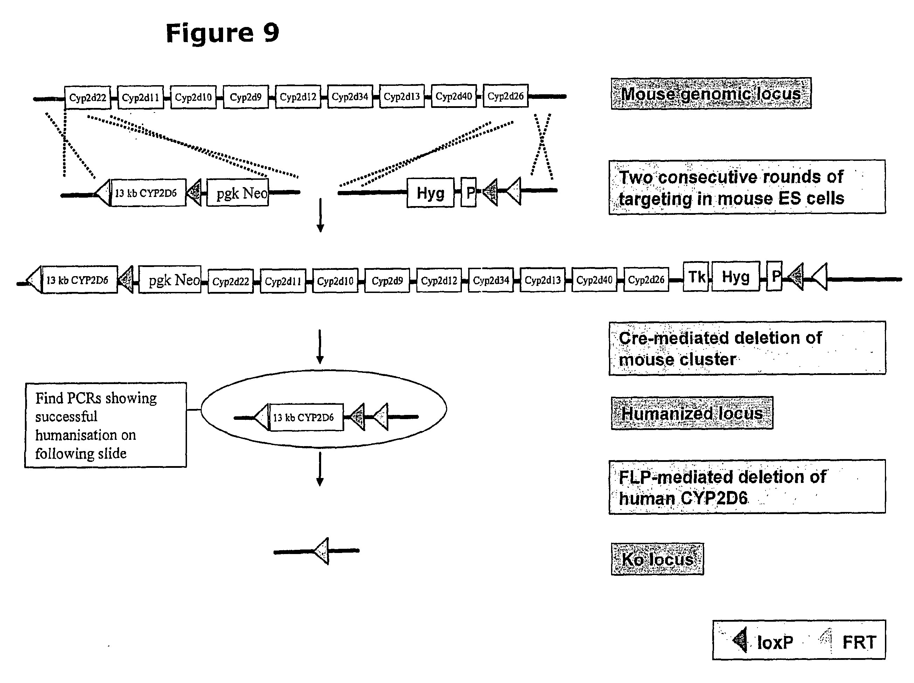

**Figure 5****. Principles of the invention if expression of the human gene is to be controlled by a human promoter wherein A) expression of mouse gene at the end of the cluster is oriented outwards, B) expression of mouse gene at the end of the cluster is oriented inwards and the promoter of this gene is defined or C) expression of mouse gene at the end of the cluster is oriented inwards and the promoter of this gene is not defined. A)** The human replacement gene sequence is inserted into the mouse genome upstream of the mouse gene and mouse promoter. Subsequent Cre-mediated recombination will lead to a physical deletion of the mouse gene(s) at the end of the cluster including the promoter sequences. This strategy was chosen at one end of the Cyp3a cluster both in case of CYP3A4 into Cyp3a11 (Figure 11) and CYP3A4 into Cyp3a25 (Figure 7) and at one end of the Cyp2d Cluster in case of the CYP2D humanisation (Figure 9). **B)** The human replacement gene sequence is inserted into the mouse genome upstream of the mouse gene and mouse promoter. Subsequent Cre-mediated recombination will lead to a physical deletion of the mouse gene(s) at the end of the cluster including the promoter sequences. However, the length of a mouse promoter is usually not well defined, therefore this strategy should not be used in the method of the present invention. **C)** The human replacement gene sequence is inserted into the mouse genome between the mouse promoter and the mouse gene. Cre-mediated recombination will therefore leave the mouse promoter intact. To avoid interference with the human expression cassette, a splice acceptor polyA (sApA) sequence terminates transcription of the mRNA. The sApA sequence can be located in intron 1 if the mouse gene, or in any downstream intron. If homologous recombination is such that it takes place within an exon of the mouse gene, a polyA signal alone (without a splice acceptor site) is sufficient to terminate the transcription. This strategy was chosen at the other end of the Cyp3aCluster both in case of CYP3A4 into Cyp3a11 (Figure 11) and CYP3A4 into Cyp3a25 (Figure 7) and at the other end of the Cyp2d Cluster in case of the CYP2D humanisation (Figure 9) and at both ends of the Cyp2cCluster in case of CYP2C9 in Cyp2c55 (Figure 8). It should be noted that the same consideration shown in A), B), and C) for the one end of the mouse cluster (shown throughout the figure at the left end) should also be made for the other end of the cluster.

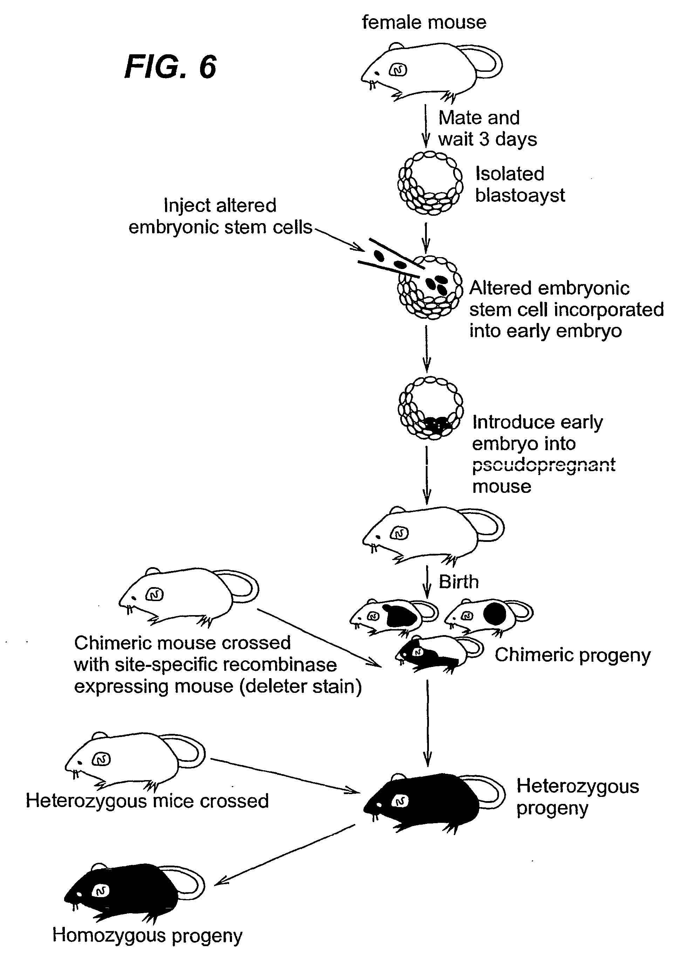

**Figure 6****. Method for the production of a transgenic mouse.** A transgenic mouse is produced by the insertion of one or more altered embryonic stem cells into a developing blastocyst. The blastocyst is then implanted into a pseudo-pregnant mouse and allowed to develop, producing a chimera. Crossing the chimera with a deleter strain effects the excision of the mouse target gene and produces mice which are heterozygous for the humanised gene. Crossing two of said heterozygotes produces mice homozygous for the humanisation event.
**Figure 7****. Method of humanisation of CYP3A4 using the corresponding human promoter** A target strategy for the generation of mice humanised for CYP3A4 wherein the mouse CYP3A cluster is deleted and the human expression cassette can be knocked out subsequently.
**Figure 8****. Method of humanisation of CYP2C9 using the corresponding human promoter** A target strategy for the generation of mice humanised for CYP2C9 wherein the mouse CYP2C cluster is deleted and the human expression cassette can be knocked out subsequently.
**Figure 9****. Method of humanisation of CYP2D6 using the corresponding human promoter** A target strategy for the generation of mice humanised for CYP2D6 wherein the CYP2D cluster is deleted and the human expression cassette can be knocked out subsequently.

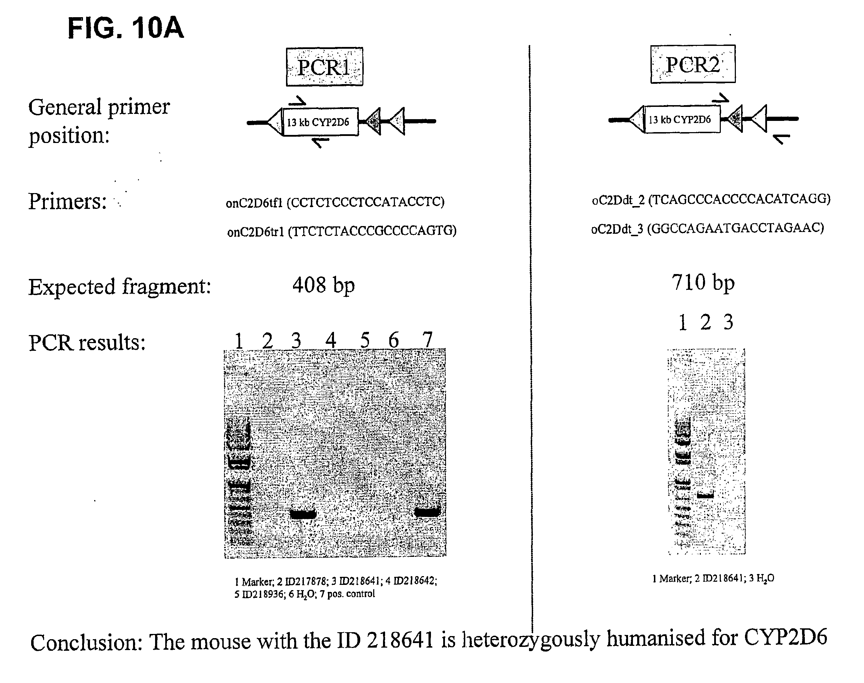

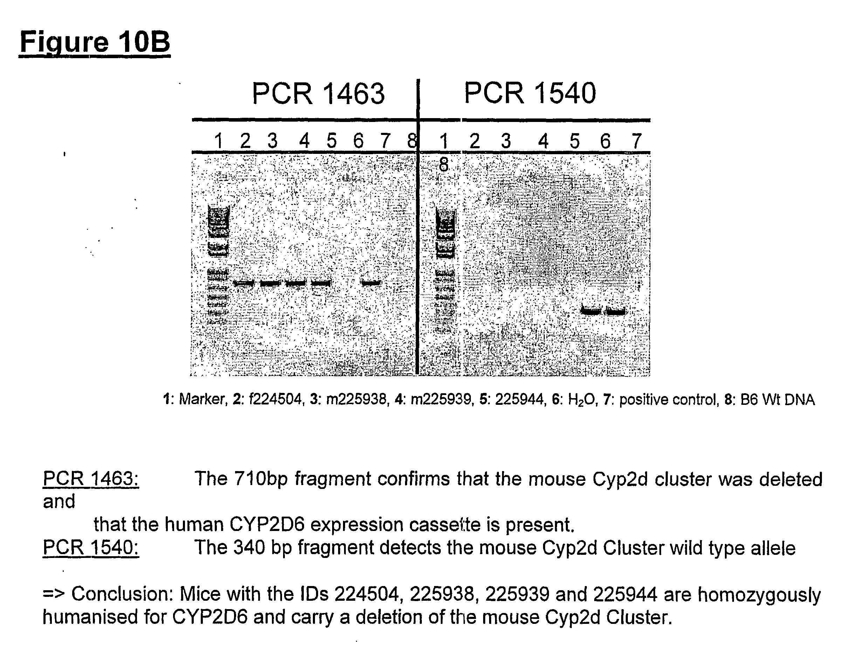

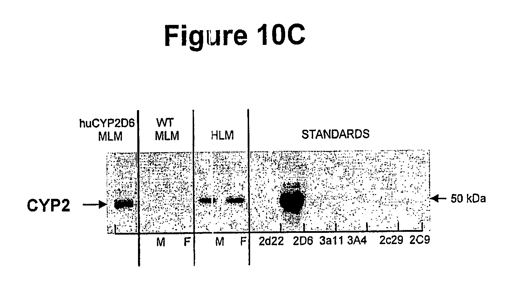

**Figure 10****. PCR analysis demonstrates successful generation of heterozygous CYP2D6 humanised mouse.** Use of specific primers allows analysis of the mouse chromosome by PCR. A): The results demonstrate successful introduction of the desired human replacement CYP2D gene sequence and the deletion of the mouse CYP2D cluster to generate a heterozygously humanised mouse for CYP2D6. B): PCR confirmation of homozygous CYP2D6 humanized mice. Mice with the IDs 224504, 225938, 225939 and 225944 are homozygously humanised for CYP2D6 and carry a deletion of the mouse Cyp2d Cluster. C): CYP2D6 protein expression in huCYP2D6 mouse liver. 1ug of male huCYP2D6 mouse liver microsomes (MLM) (n=1), along with pooled wild type mouse (n=20) and human liver microsomes (HLM) (n=20) (both sexes), were loaded onto a 10% Nu-PAGE gel. The membranes were incubated with a monoclonal mouse human-specific CYP2D6 antibody (1:2000, Gentest) and anti-mouse HRP conjugate (1:2000, GE Healthcare). ECL development with 30sec exposure. Standards loaded were are follows; 1, mouse recombinant Cyp2d22 his-tagged (0,01pmol, CXR Biosciences). 2, human CYP2D6 baculosomes (0.01pmol, Invitrogen), 3, mouse recombinant Cyp3a11his-tagged (0.1pmol, CXR Biosciences), 4, human CYP3A4 baculosomes (0.1pmol, Invitrogen), 5, mouse recombinant his-tagged Cyp2c29 (0.1pmol CXR Biosciences), 6, human CYP2C9 baculosomes (0.5pmol).
**Figure 11****. Method of humanisation of huCYP3A4 using the mouse Cyp3a11 promoter** A target strategy for the generation of mice humanised for CYP3A4 using the Cyp3a11 promoter wherein the mouse CYP3A cluster is deleted.

### Detailed description of preferred embodiments

The present invention provides a method of humanising a mouse for a gene of interest, comprising:
a) incorporating a pair of site-specific recombination sites into the mouse chromosome by homologous recombination such that the mouse target gene sequence that is to be replaced is flanked on each side by a recombination site;
   wherein at least one of the two recombination sites is constructed so as to be contiguous with a human replacement gene sequence;
   and said human replacement gene sequence is positioned so that said recombination site lies between said human replacement gene sequence and said mouse target gene sequence;
b) effecting recombination between the site-specific recombination sites such that the human sequence is introduced at the position in the mouse chromosome previously occupied by the mouse target gene, flanked by the residual site-specific recombination site.

The terms "humanise, humanised" and "humanising" as used herein relate to the introduction of a human gene or genes into the genome of a mouse, in place of the endogenous murine gene or genes. The terms can be used to describe the effect of such gene replacement on a mouse chromosome, cell, tissue, organ or organism.

### Methodology

According to the present invention, replacement human genes that are inserted into the transgenic model are preferably inserted at the point in the chromosome where the endogenous equivalent gene or gene cluster naturally occurs. This has the advantage that the context of the gene locus is retained which means that the fidelity of transcription from this site is as close as possible to the level of transcription that occurs in the wild type system.

The recombination steps are preferably performed in a mouse embryonic stem cell, according to methods well known in the art and discussed further below. Embryonic stem cells are cultured cell lines of totipotent cells, wherein the cells, when introduced into an early embryo, will develop to populate all tissues of the developing organism.

The first step in the method is the incorporation of a pair of site-specific recombination sites into the mouse chromosome, also called recombinase target or RT sites. RT sites are recognised by site-specific recombinases (SSRs). Exposure to SSR enzyme activity results in a DNA rearrangement determined by the disposition of the RT sites, which in a linear DNA molecule results in the intervening sequence being excised, or cut out.

The mouse target gene sequence to be replaced should be flanked on each side by an RT site. Recombination between the two RT sites will thus cut out the intervening sequence.

One or both of the two recombination sites is designed so as to be contiguous with a human replacement gene sequence. The human sequence should be positioned so that the recombination site lies between the human replacement gene sequence and the mouse target gene sequence that is to be replaced. This has the effect that when recombination occurs between the two RT sites, so cutting out the mouse target sequence, the human sequence is introduced at the position in the mouse chromosome previously occupied by the mouse target gene. A residual site-specific recombination site will remain at the point where the mouse target sequence previously resided. Advantageously, the integration event is designed such that the residual RT site does not interrupt the coding sequence of the introduced gene or adversely influence transcription from the governing promoter. This can be achieved either by careful placement of the RT sites so as not to perturb the coding sequence or by placement of the RT sites within introns.

In one embodiment of the invention, more than one human replacement gene sequence may be used, such that a human replacement gene sequence is positioned both upstream and downstream of the endogenous mouse target gene sequence. In this manner, upon site-specific recombination, the mouse target sequence is excised and the replacement human sequences are brought together in its place.

There are a number of situations in which such a strategy might be advantageous. For example, given the size limitations of existing homologous recombination techniques, it might be necessary to incorporate two separate human flanking replacement sequences in order to introduce the amount of replacement sequence that is desired. For example, CYP1A1 might be introduced upstream of the endogenous murine equivalent, whilst CYP1A2 could be introduced downstream.

It also might be appropriate to introduce two separate genes in this manner, perhaps genes that are too far apart in a gene cluster, so that they are not covered by a single BAC or YAC, or two genes that are located on different chromosomes. Such a strategy could not practically be performed using prior art systems.

The invention also allows the formation of a functional human gene by combining two component halves that are introduced either side of a mouse gene or cluster on the chromosome. Upon site-specific recombination to excise the mouse gene or cluster, the component halves of the human gene are brought together so as to form a functional human gene. By this means, a time or tissue specific activation of the human gene and inactivation of the mouse gene(s) could be achieved. The potential problem of the residual RT site disrupting coding sequence can be resolved by placement of this RT site within an intron.

Methods for incorporation of the RT sites into the chromosome will be known to those of skill in the art, and is preferably performed by exploiting the process of homologous recombination. Homologous recombination relates to the genetic mechanism which can be exploited to allow the insertion a nucleic acid sequence into the mouse chromosome. The mechanism is initiated by the alignment of double-stranded mouse and human nucleic acid sequences. A double strand break in the mouse sequence and 5' to 3' exonuclease activity facilitate strand invasion, resulting in pairing of the homologous human and mouse sequences through short regions of homology. Subsequent chain elongation of the mouse sequence utilises the human sequence as a template and resolution produces the mouse genomic sequence with the human sequence located within it, whilst the human sequence remains intact.

Methods for performing homologous recombination are known in the art and exploit regions of homology between exogenously supplied DNA molecules and the target chromosome to introduce the RT sites. Examples of suitable targeted delivery systems will be clear to those of skill in the art and include the use of injected or targeted naked DNA, targeted liposomes encapsulating and/or complexed with the DNA, targeted retroviral systems and targeted condensed DNA such as protamine and polylysine-condensed DNA, or electroporation. Other delivery methods may also be employed, such as by using nucleic acid expression vectors, polycationic condensed DNA or ligand linked DNA (see Curiel (1992) Hum Gene Ther 3:147-154; Wu (1989) J Biol Chem 264:16985-16987), and use of a gene transfer particle gun, (described in US 5,149,655). Naked DNA may also be employed, as is described in detail in International patent application WO 90/11092.

The RT pair is preferably selected from the group consisting of LoxP, FRT, attP/attB and rox (Sauer and McDermott, Nucleic Acids Res. 32(20): 6086-95, 2004). This list is provided by way of example only, and is not intended to be limiting. Within this aspect recombination between said site-specific recombination site pairs is to be effected by the corresponding site-specific recombinase, i.e. Cre, FLP, PhiC31 and Dre respectively. It is to be understood that said site-specific recombination can be effected *in vivo* or *in vitro*.

As a practical matter, each RT site should be constructed so as to be linked to, and preferably contiguous to a selectable marker. The inclusion of a selectable marker allows the detection of the RT site within the mouse chromosome, and as such allows monitoring for the successful insertion of RT site pair.

Each selectable marker is preferably positioned so that it lies in the space between the mouse target sequence and the RT site. This means that upon successful recombination, the selectable markers are removed from the chromosome and thus take no further part in the methodology. In this way, it can be sure that the selectable marker has no perturbing effect on the regulation of the human sequence that remains in the chromosome after humanisation.

Each of the selectable markers are preferably different to each other. This allows the insertion of each recombination site to be monitored separately and independently from each other.

The selectable markers are preferably genes encoding some kind of resistance to a chemical compound to which the growing ES cells can be exposed, such as an antibiotic. Examples include use of selectable markers conferring resistance to antibiotics added to the growth medium of cells, for instance the neomycin resistance marker conferring resistance to G418, hygromycin or puromycin. Further examples involve detection using nucleic acid sequences that are of complementary sequence and which will hybridise with, or a component of, the nucleic acid sequence in accordance with the previous aspects of the invention. Examples would include Southern blot analysis, northern blot analysis and PCR.

A preferred strategy involves the creation of altered murine embryonic stem cells, preferably in separate steps, whereby each RT site is incorporated separately, with its selectable marker. The altered embryonic stem cell may be subsequently inserted into a blastocyst. Conventionally, blastocysts are isolated from a female mouse about 3 days after it has mated. It is to be understood that up to 20 altered embryonic stem cells may be simultaneously inserted into such a blastocyst, preferably about 16. Through insertion of altered embryonic stem cells into the blastocyst, the embryonic stem cell will become incorporated into the developing early embryo, preferably by its transplantation into a pseudo-pregnant mouse which has been induced so as to mirror the characteristics of a pregnant mouse. According to this methodology, the blastocyst, containing the altered embryonic stem cell, will implant into the uterine wall of the pseudo-pregnant mouse and will continue to develop within the mouse until gestation is complete. The altered embryonic stem cell will proliferate and divide so as to populate all tissues of the developing transgenic mouse, including its germ-line.

In one aspect of the methodology, the created transgenic mouse may be a chimera, containing altered and non-altered cells within each somatic tissue and within the germ-line.

In order to effect recombination between the RT sites, the genome must be exposed to SSR activity, in the form of an SSR enzyme. The term "SSR" refers to any protein component of any recombinant system that mediates DNA rearrangements in a specific DNA locus, including SSRs of the integrase or resolvase/invertase classes (Abremski, K.E. and Hoess, R.H. (1992) Protein Engineering 5, 87-91; Khan, et al., (1991) Nucleic acids Res. 19, 851-860; Nunes-Duby et al., (1998) Nucleic Acids Res 26 391-406; Thorpe and Smith, (1998) P.N.A.S USA 95 5505-10) and site-specific recombination mediated by intron-encoded endonucleases (Perrin et al., (1993) EMBO J. 12, 2939-2947).

Preferred recombinase proteins are selected from the group consisting of: FLP recombinase, Cre recombinase, Dre recombinase, R recombinase from *Zygosaccharomyces rouxii* plasmid pSR1, a recombinase from the *Kluyveromyces drosophilarium* plasmid pKD1, a recombinase from the *Kluyveromyces waltii* plasmid pKW1, TrpI from the Bacillus transposon Tn4430, any component of the λ Int recombination system, phiC31, any component of the Gin recombination system, or variants thereof.

The methodology for mediating Cre/lox-mediated deletions, suitable for deleting of large fragments of DNA (200kb to several megabases), has been described in the following papers ( Li ZW, Stark G, Gotz J, Rulicke T, Gschwind M, Huber G, Muller U, Weissmann C. Generation of mice with a 200-kb amyloid precursor protein gene deletion by Cre recombinase-mediated site-specific recombination in embryonic stem cells Proc Natl Acad Sci USA. 1996 Jun 11;93(12):6158-62. Erratum in: Proc Natl Acad Sci U S A 1996 Oct 15;93(21):12052; in Su H, Wang X, Bradley A. Nested chromosomal deletions induced with retroviral vectors in mice. Nat Genet. 2000 Jan;24(1):92-5); Call LM, Moore CS, Stetten G, Gearhart JD. A cre-lox recombination system for the targeted integration of circular yeast artificial chromosomes into embryonic stem cells. Hum Mol Genet. 2000 Jul 22; 9(12):1745-51).

As mentioned above, the site-specific recombination event may be effected *in vitro* or *in vivo*. Site-specific recombination may be effected by inducing activity of the SSR within the transgenic mouse. Indeed, successful exploitation of site-specific recombination to alter genotype in living systems requires strategies to regulate the recombination event. This can be done by controlling expression of the recombinase mRNA, or protein (Baubonis and Sauer (1993) Nucl Acids Res. 21, 2025-2029; Sauer B, (1994) Curr Opin Biotechnol 5:521-7; Rajewsky et al., (1996) J Clin Invest 98, 600-3; Metzger and Feil, (1999) Curr. Opinions Biotechnology 10, 470-476), such that the expression pattern achieved is confined to the times and places at which these tissue specific elements are active.

Researchers have used direct transfection, infection with recombinant viruses or injection of the DNA or mRNA encoding SSR protein or the protein itself (Konsolaki et al., (1992) New Biol. 4: 551-557) in order to express SSR enzymes. A more precise degree of control may be attained by regulating the activity rather than the expression of these SSR enzymes. One strategy uses fusion proteins in which a SSR enzyme is fused to the ligand binding domain (LBD) of a steroid receptor to give an SSR-LBD protein (see EP-B-0 707 599; also Logie and Stewart (1995) P.N.A.S. USA 92: 5940-5944; Brocard et al., (1997) P.N.A.S. USA 94: 14559-14563; Akagi et al., (1997) Nucleic Acids Res 25, 1766-73). This strategy relies on the application of a ligand for the steroid receptor that activates the SSR activity only when ligand is bound to the receptor moiety. The LBD of the receptor represses the activity of the SSR in the absence of a cognate ligand. Delivery of the cognate ligand relieves repression of the SSR, thus permitting recombination between RT sites.

Induction may thus be effected by inducing transcription of said SSR, inducing translation of the SSR, or removal an inhibitor from the SSR. Alternatively, an SSR may be artificially introduced into said transgenic mouse. One element of the methodology is that site-specific recombination can be effected within the transgenic mouse, so resulting in the excision of the mouse target gene and the concomitant production of a humanised mouse.

Preferably, site-specific recombination can be effected by crossing the transgenic mouse with a deleter strain mouse. The term "deleter strain" as used herein relates to a mouse expressing the site-specific recombinase in the germline, which can be crossed with a transgenic mouse to effect excision of the mouse target gene sequence. In this manner, *in vivo* recombination produces offspring heterozygous for the gene of interest. Crossing the transgenic mouse with a deleter strain will thus result in the production of progeny, with cells containing the mouse chromosome altered to contain the human replacement gene sequence and the site-specific recombinase, resulting in the excision of the mouse target gene and the functional humanisation of the cells. Such a transgenic mouse will therefore be heterozygous for humanisation of the specific gene or cluster of genes. Within a further aspect of the methodology, the site-specific recombinase may only be expressed in a certain tissue of the recombinase strain mouse. It is known in the art that deletion of certain genes or clusters of genes may be lethal or may have sublethal phenotypic effects. Furthermore, replacing such genes with their human equivalents may not prevent lethality. In these circumstances, it may be possible to overcome any such problems of lethality by expressing the site-specific recombinase only in certain tissues. This will be particularly advantageous if a specific gene is known to be essential in a certain tissue, as expression of the site-specific recombinase in this manner allows the mouse gene to persist in those tissues.

Within this aspect of the invention, the SSR may be albumin-Cre. Albumin-Cre is a specific variant of the SSR Cre which acts on the RT site LoxP. Albumin-Cre is expressed only in the liver, and will therefore allow the mouse target sequence to persist in all tissues except the liver, overcoming possible problems of lethality, whilst providing a functionally humanised liver.

Ultimately, two heterozygous mice produced according to the methodology above may be crossed to produce a transgenic mouse that is homozygous for the human allele of the gene or genes of interest. Crossing two heterozygous transgenic mice will produce a proportion of progeny that are homozygous for the humanisation.

In a further embodiment of the invention the transgenic non-human animal is produced *de novo* so as to include all of the aforementioned features, by the methods as hereinafter disclosed.

It is also possible that the site-specific recombination event be effected in a somatic cell which could then be used as a nuclear transfer donor cell in order to make a colony of cloned mice according to the methodology of WO00/51424 or a variation thereof.

In embodiments of the disclosure relating to the preparation of a transgenic host cell or a transgenic non-human animal comprising the use of a nucleic acid construct as previously described, the cell or non-human animal may be subjected to further transgenesis, in which the transgenesis is the introduction of an additional gene or genes or protein-encoding nucleic acid sequence or sequences. The transgenesis may be transient or stable transfection of a cell or a cell line, an episomal expression system in a cell or a cell line, or preparation of a transgenic non-human animal by pronuclear microinjection, through recombination events in non-embryonic stem (ES) cells, random transgenesis in non-human embryonic stems (ES) cells or by transfection of a cell whose nucleus is to be used as a donor nucleus in a nuclear transfer cloning procedure.

Methods of preparing a transgenic cell or cell line, or a transgenic non-human animal, in which the method comprises transient or stable transfection of a cell or a cell line, expression of an episomal expression system in a cell or cell line, or pronuclear microinjection, recombination events in ES cells, or other cell line or by transfection of a cell line which may be differentiated down different developmental pathways and whose nucleus is to be used as the donor for nuclear transfer; wherein expression of an additional nucleic acid sequence or construct is used to screen for transfection or transgenesis in accordance with the previous aspects of the invention. Examples include use of selectable markers conferring resistance to antibiotics added to the growth medium of cells, for instance neomycin resistance marker conferring resistance to G418. Further examples involve detection using nucleic acid sequences that are of complementary sequence and which will hybridise with, or a component of, the nucleic acid sequence in accordance with the previous aspects of the invention. Examples would include Southern blot analysis, northern blot analysis and PCR.

### The human replacement gene sequence

The invention may be implemented using any one of a number of genes, as will be clear to those of skill in the art. There is no technical limitation to the type of gene that may be exchanged between mouse target and human replacement. However, the invention lends itself particularly to genetic systems in which the mouse functionality is coded for by a number of different genes, linked contiguously in the chromosome in a cluster. The method allows the simple replacement of the mouse gene cluster for the equivalent human gene or cluster. The method is also suited to humanisation of a mouse genetic system which contains a high level of redundancy at the genetic level. In contrast to methods that have been used previously, there is no requirement or preference for the exchanged gene(s) to be a segmented gene, such as an immunoglobulin gene; in preferred embodiments, the exchanged gene(s) is not a segmented gene, such as an immunoglobulin gene.

Preferably, therefore, the expression product of at least one mouse target gene retains the same, similar or identical function as the equivalent human replacement gene. The genes may be functionally equivalent, and/or structurally homologous. For example, the mouse target gene and human replacement gene may share a degree of homology. Preferably, such homology will be greater than 30%, greater than 40%, greater than 50%, greater than 60%, greater than 70%, greater than 80%, greater than 90%, or even greater than 95%.

The human replacement gene sequence may comprise cDNA, genomic DNA, or a mixture of the two. Genomic DNA is advantageous in many circumstances because the fidelity of splicing will be retained. However, it may only be necessary to retain those introns where the majority of splice events take place, such that the remainder of the sequence can be cDNA. This can simplify the cloning process, particularly where the genomic DNA comprises large introns; in such cases the larger introns may not be included provided that splice isoforms are not coded for in this area of the genomic DNA.

Examples of particularly suitable genes for use in the method of the present invention are drug-metabolising enzyme genes, including, for example, phase-1 drug-metabolising enzymes, phase II drug-metabolising enzymes, and transcription factors. Examples include the *cyp* enzyme family, a family of hemoproteins which contain a heme iron centre and oxidise a variety of substrates through a system of electron transport. The heme centre of *cyp* protein is reduced by electrons passed from cytochrome P450 reductase, ferredoxins, or cytochrome b5, molecular oxygen is bound and split by the reduced heme group and the substrate is oxidized to an alcohol or an epoxide, with regeneration of the resting Fe³⁺ state of the heme group. A review of such proteins, that compares human and murine CYP genes is provided by Nelson et al., 2004 (Pharmacogenomics 14(1); 1-18). Other examples will be clear to those of skill in the art.

Preferably, the human DNA encoding a phase-1 drug-metabolising enzyme is selected from the group comprising the cytochromes P450, including but not limited to CYP3A4, CYP2B6, CYP2C9, CYP2C19, and CYP2D6. In this example, equivalence between genes can be assessed by a combination of substrate specificity, mode of regulation (for example, by transcription factors or exogenous drugs), sequence homology and tissue distribution. Certain genes have exact equivalents; examples of such genes are CYP2E1, CYP1A1, CYP1A2, CYP2B6 and CYP2D, where there is only one gene in the human, but numerous equivalent genes in the mouse. There are four CYP2C genes in the human, and numerous equivalent genes in the mouse. In such circumstances, at least one, preferably two, three, four, five or more or even all of the equivalent murine genes are annulled. CYP3A4 is an example where there is no obvious orthologue in the mouse, but CYP3A11 could be considered at least one equivalent mouse gene because of its hepatic expression, mode of regulation and sequence homology. Another example can be provided by the case of CYP2D6, where there are nine genes in the mouse, corresponding to only one functional gene in humans.

The choice of human P450 isoforms for introduction into P450-humanised non-human animals is predominantly driven by the known relative importance of various P450 isoforms in metabolism in the relevant tissue. Thus, for example, to date the single most significant P450 isoform recognised in the human liver is CYP3A4, and so CYP3A4 is therefore probably the first human P450 isoform of choice for P450 humanisation of liver. The choice of human P450s for the multi-P450-humanised mouse of the present invention is dictated by the need of the user. In this respect it is expected that any one or more of the following human isoforms will be preferred: 3A4, 3A5, 2D6, 2B6, 2C9, 2C19, 1A1, 1A2, 2C8, 2E1. However, it will be appreciated that the isoform(s) incorporated into the animal cell is/are dependent on the user's requirement. In this way, the humanised transgenic animal may be "designed" to investigate the role of specific isoforms in the metabolic process.

According to the teaching of the invention, single genes, gene clusters or combinations of single genes or gene clusters may be replaced. Whole gene clusters should preferably be replaced where possible, rather than simply replacing individual genes. This generates a situation in which the ratios of the expression levels of genes in any gene cluster are the same as the ratios in which these genes are expressed *in vivo.* The introduced cluster of genes is intended to be functionally equivalent to the mouse target gene, or, where there are equivalent gene clusters, it is equivalent to the mouse target gene cluster. Replacing the appropriate mouse target gene(s) with the equivalent human replacement gene sequence(s) will therefore result in the humanisation of the genome, cell, tissue, organ or organism regardless of whether the human or mouse cluster contain the same number of genes.

The term "cluster" as used herein is thus any region of the genome which has the ability to generate more than two gene products, and includes multiple splicing of the same gene. Within this aspect, the gene cluster may preferably encode proteins involved in metabolism, and more preferably may form part of the cytochrome P450 (*cyp*) gene cluster. For example, there are considered to be seven P450 gene clusters in the mouse; the CYP2ABFGST cluster, the CYP2C cluster, the CYP2D cluster, the CYP2J cluster, the CYP3A cluster, the CYP4ABX cluster and the CYP4F cluster. The CYP3A, CYP2C and CYP2D clusters are preferred clusters of phase-1 drug-metabolising enzymes for replacement according to the present invention. Any one or more of these gene clusters may be replaced according to the present invention.

According to the invention, therefore, partial, or preferably complete cascades of genes that are implicated in a particular pathway may preferably be replaced. This ensures that the partial redundancy of gene function is retained and so, again, the real physiological situation is mirrored. Preferably, the clusters of phase 1 drug metabolising enzymes that are used for humanisation are the CYP3A cluster and the CYP2C cluster.

In view of the redundancy in protein function between the human and commonly used target transgenic animals such as the mouse, humanisation for phase 1 drug metabolising enzymes is preferably performed against a deleted background in which only some (for example 1, 2, 3, 4 or 5), and preferably none of the target animal phase 1 drug metabolising enzymes are expressed at significant levels.

Other examples of human drug metabolising enzymes that are candidates for humanisation according to the invention include the glucuronyl transferases, for instance, the UGT1A gene or gene cluster, the glutathione transferases, for instance GST (glutathione S-transferases), the sulphonyl transferases and the acetyl transferases. Preferably, a cluster of phase 2 drug metabolising enzymes that is used for humanisation is the UGT1A gene cluster.

### Regulatory sequences

The regulatory sequences governing expression of the transcription factor(s) may preferably be either of human origin, or may originate from the target animal species e.g. the mouse.

In the majority of circumstances it will be desired to use the human regulatory sequences. This can be effected simply by including these within the human replacement gene sequence that is inserted into the mouse chromosome by homologous recombination. The RT site is thus inserted such that the mouse target sequence, flanked by both the RT sites, includes the endogenous mouse promoter(s) which is subsequently excised upon site-specific recombination.

As shown in Figure 5, several factors must be taken into account when deciding upon the use of human regulatory sequences. These include the orientation of the mouse genes at the edge of the cluster and the availability of information on the mouse promoters and enhancer elements. However, there is generally a mechanism by which the human promoter can be functionally inserted into the mouse genome to control expression of the humanised gene(s).

In one advantageous embodiment that is preferred when using the human regulatory sequences, particularly when inserting a replacement gene into an area of the mouse chromosome that is transcriptionally active, an artificial polyadenylation sequence can be fused to a downstream exon of any gene(s) upstream of the inserted human sequence. For example, a splice acceptor polyadenylation (sApA) site can be used in this context, similar to the use of this sequence as described by Ishida and Leder (Nucleic Acids Res. 27(24), e35, 1999). This ensures that there is no read-through from transcriptional activity upstream of the introduced human replacement gene and regulatory sequence, which allows precise study of the human sequence, without influence from other promoter sequences.

Under some circumstances, insertion of the human gene(s) at the position in the mouse chromosome previously occupied by the mouse target gene may allow the endogenous regulatory sequences of the mouse gene to be used, provided that suitable endogenous regulatory sequences are located in the required orientation at the edge of the mouse cluster, as shown in Figure 4.

In some circumstances in which the invention is likely to be exploited, the endogenous murine regulatory sequences will be used. In this way, the humanised animals made according to the present invention are able to express the human proteins at not only the appropriate physiological levels but in all tissues in which the mouse would normally express the equivalent endogenous protein(s). However, it should be noted that the murine regulatory sequences in some cases might confer a more human like expression to a human gene in the mouse than the corresponding human promoter. For example, though CYP3A4 is expressed constitutively in the liver of adult men, in transgenic mice the CYP3A4 promoter seems to be silenced in the liver of males older than six weeks. Therefore, using the murine Cyp3a11 promoter, which shows similar hepatic expression and regulation as the CYP3A4 promoter in humans, to drive the expression of human CYP3A4, might give a more bona fide expression of this gene in mice.

In this embodiment, where an endogenous mouse promoter is known to be present at the correct location and orientation to drive expression of the human replacement gene sequence, the RT sites should be inserted into the mouse chromosome so that the upstream one of the pair of RT sites lies between the mouse target gene and the endogenous mouse promoter of the mouse target gene. Upon site-specific recombination, the endogenous mouse promoter remains within the chromosome becomes linked transcriptionally to the replacement human gene sequence, so as to effect transcription of the human replacement gene sequence, as shown in Figure 4A. By "regulatory genes" is meant to include any promoter or enhancer sequences, 5' or 3' UTRs, poly-A termination sequences or other DNA sequences, that are necessary for transcription of the gene of interest. Transcripts used for insertion of human sequences are preferably terminated by a poly A motif.

By "endogenous promoter" is meant the promoter that naturally directs expression of the gene of interest in the mouse.

For both the endogenous and human promoter sequences, incorporation of all of the 5' upstream sequence that is necessary for promoter activity, preferably including any enhancers, has shown that it is not in fact necessary and often not desired to use strong, constitutive promoters; as in the natural situation, the endogenous promoter, or the human inducible promoter in its entirety, is perfectly capable of directing expression of the relevant protein in a physiologically relevant manner. An example is provided by the human CYP1A2 gene, which possesses strong enhancer elements upstream of the transcription initiation point. Whilst incorporation of all this human sequence allows the appropriate mechanism of transcription to occur, omission of these upstream sequences leads to a system in which incomplete or insufficient regulatory sequences are present to allow the fidelity of gene expression to be retained as shown by Chen et al. (Journal of Pharmacology and Experimental Therapeutics 318, 1330-1342, 2006).

One disadvantageous result of using promoters such as the albumin promoter that have been used in the prior art, certainly in the case of drug metabolising genes such as CYP3A2, is that the effects of expression of a gene can only be monitored in a particular tissue, in the case of the albumin promoter, this is the liver. Of course, many genes have quite diverse patterns of expression, and these are often little researched. Use of the endogenous mouse promoter or the corresponding human promoter avoids all such problems, and guarantees that the human gene, if properly configured and aligned within the chromosome, will retain the fidelity of the level of wild type murine expression, developmentally, temporally and in a tissue-specific manner. As a result, the human protein is produced in the correct place, at the correct level, at the correct time and for the appropriate amount of time. In the case of a mouse humanised for CYP3A2, therefore, the invention allows a global, holistic snapshot to be obtained of the drug metabolism process.

Use of the endogenous promoter or the corresponding human promoter also carries other advantages with it. In particular, the fidelity of developmental expression is retained. The use of the natural endogenous promoter ensures that the protein is expressed in the tissues in which it naturally occurs, and not only in the adult animal, but also at each developmental stage. This also carries with it the advantage that the transgenic animals are more likely to be viable and thus useful as screens and in the development of downstream crosses. In the case of drug metabolism genes, it also allows the animals to be used to screen for teratogenic effects of a test compound, as placental expression of drug metabolising enzymes is retained. In some circumstances, the target gene and the human replacement gene sequence may share a leader sequence. This may be achieved by retaining at least one intron in the construct, which usually results in a better expression. This strategy also ensures that the gene product will be guided to the right intracellular location. The human leader sequence might be able to fulfil that function as well, but it is often safer to use the mouse leader instead. For instance, in the case of the MRP2 protein, the "leader sequence" of the mouse protein, which is encoded by exon 1, may be retained. The human cDNA without sequences from exon 1 is then introduced into exon 2 of the mouse genomic sequence. The original splice sites for mouse intron 1 will be retained, so that this construct encodes a fusion protein of amino acids from mouse exon 1 and human exons 2-32. This construct ensures a high level of expression and also that the MRP2 is guided to its correct location, the plasma membrane.

The skilled person will also understand how the human replacement gene sequence may be brought under control of the promoter of the target gene in the mouse so as to be linked transcriptionally to that promoter. By "linked transcriptionally" is meant that the activity of the promoter dictates the expression level of the corresponding protein. Preferably, this is achieved by fusing the human replacement gene sequence to the translational start site of the corresponding mouse promoter. For example, in the case of the multidrug resistance protein MDR1, the cDNA of human MDR1 may be fused to the translational start site of the corresponding mouse genes (*Mdr1a* or *Mdr1b*)*.* In the example of the transcription factor PXR, a hybrid of human PXR cDNA and genomic sequences may be fused to the translational start site of the mouse PXR gene, whereby the mouse Start-ATG is retained. In the case of another human transcription factor CAR, the human sequence may be fused to the translational start site of the mouse CAR gene.

### Reporter genes

The present invention also, advantageously provides non-human animal cells and transgenic non-human animals incorporating introduced reporter genes so that such cells or animals can be used to determine indications of pathways of metabolism of drugs or other xenobiotic compounds in a human cell by convenient assay of the products of reporter gene expression.

Where reporter genes have been incorporated in non-human animal cell or transgenic non-human animals produced by the method of the invention (see below), the cells or animals can be used to determine regulation of genes and also give indications of the likely mechanism and metabolism of drugs or other xenobiotic compounds in an homologous human cell by assaying expression of the reporter gene DNA sequence. The cells or animals can also be used to give indications of the extent of metabolism of drugs or other xenobiotic compounds. For example, analysis of the distribution of reporter gene expression within any particular tissue allows the extent of induction of gene expression to be monitored in response to a particular drug compound.

Reporter genes are nucleic acid sequences encoding directly or indirectly assayable proteins. They are used to replace other coding regions whose protein products are unsuitable or not amenable to the assay envisaged. Suitable reporter genes that are known in the art and may be used in the present invention are selected from those genes encoding proteins including but not limited to: chloramphenicol-acetyltransferase, β-galactosidase, β-glucuronidase, luciferase, beta-galactosidase, green fluorescent protein, secreted alkaline phosphatase (SEAP), major urinary protein (MUP) or human chorionic gonadotrophin (hCG). It will be understood that the above list of suitable reporter genes is not exhaustive or exclusive and is not intended to limit the scope of the application. The skilled artisan may select another reporter system which will equally be applicable to the present invention.

According to the invention, the mouse promoters that are preferred targets for linkage to reporter genes are PXR, CAR, CYP3A4, CYP2C9, CYP2C19, CYP2B6, CYP2D6, UGT1A, MRP2 and MDR1.

### Additional site-specific recombination sites

In a further embodiment of the invention, a second pair of RT sites may be incorporated into the mouse chromosome, again preferably by homologous recombination. This second pair of RT sites should be positioned so that both component RT sites flank the first pair of RT sites, as shown in Figure 1. This second pair of RT sites allows the human replacement gene sequence to be excised should this be desired. This allows a set or panel of transgenic mice to be generated whose properties can be compared; the full set may thus comprise wild type, humanised knock-in and complete knock-out. Furthermore, for certain applications, mice heterozygous for knock-ins or knock-outs can also be compared in the panel.

The second pair of RT sites may be inserted into the mouse chromosome simultaneously, prior to or subsequent to effecting RT between the first pair of site specific recombination sites. Preferably, the two pairs of sites are inserted simultaneously, for convenience of engineering and cloning.

Following site-specific recombination between the first pair of RT sites, the second pair of RT sites will be positioned in the chromosome flanking the human replacement gene sequence. In this configuration, the residual RT site produced during recombination between the first pair of RT sites, will be positioned between one component of the second pair of site specific recombination sites and the human replacement gene sequence, as shown in Figure 1.

Within this aspect it is intended that a second round of RT may be effected between the second pair of RT sites such that the human replacement gene sequence is excised and the gene of Interest is knocked out. Herein, the residual first RT site will also be excised, whilst the residual second RT site remains.

The second pair of RT sites and first pair of RT sites may be incorporated into the mouse chromosome either concurrently or separately.

Wherein the second pair of RT sites is to be incorporated concurrently with the first pair of RT sites, one component of each pair will preferably be contained within a single nucleic acid sequence, which can be incorporated into the mouse chromosome through one round of homologous recombination. The other component of each pair may be contained within a second nucleic acid sequence, which is also incorporated into the mouse chromosome, through a second round of homologous recombination. Each component of the second pair of RT sites is positioned in the chromosome such that one half of the first pair of RT sites is positioned between the half of the second pair of RT sites and the selectable marker. These two rounds of homologous recombination may take place simultaneously or separately.

Wherein the second pair of RT sites is to be incorporated into the mouse chromosome separately to the first pair of RT sites, each half of the second pair of RT sites is preferably contained within a separate nucleic acid sequence, and the second pair of RT sites is incorporated into the mouse chromosome through a further two separate homologous recombination reactions, through the mechanism shown in Figure 2. These two further rounds of homologous recombination may take place simultaneously or consecutively with respect to each other and with respect to the first two round of homologous recombination. It is to be understood that the recombination reactions are intended to be performed either *in vivo* or *in vitro.*

It is preferred that the first and second pairs of RT sites are different from each other.

### Transgenic mice

Here is further disclosed a transgenic mouse produced by a method of any one of the embodiments of the invention described above. Such a mouse is humanised for a gene or gene cluster and the corresponding mouse gene or cluster has been deleted.

Various aspects and embodiments of the present invention will now be described in more detail by way of example. It will be appreciated that modification of detail may be made without departing from the scope of the invention.

### Examples

### Example 1: CYP3A4 humanisation using the corresponding human promoter and knockout of the mouse CYP3A cluster

The mouse CYP3A cluster was flanked with loxP and FRT sites and a CYP3A4 expression cassette was inserted at one end of the mouse cluster, as shown in Figure 7, enabling expression of human CYP3A4 under the control of the 13 kb human CYP3A4 promoter.

The loxP sequence elements included in the targeting vectors enable Cre-mediated deletion of the mouse CYP3A cluster, to allow the expression of CYP3A4 in the absence of the corresponding mouse genes (see Figure 7).

Subsequently, the FRT sequence elements enable Flp-mediated deletion of the human expression cassette, resulting in a complete knockout at the mouse CYP3A locus (Figure 7).

### Example 2: CYP2C9 humanisation using the corresponding human promoters and knockout of the mouse CYP2C cluster

The mouse CYP2C cluster was flanked with loxP and FRT sites and a CYP2C9 expression cassette was inserted at one end of the mouse cluster, as shown in Figure 8, enabling expression of human CYP2C9 under the control of the 12 kb human CYP2C9 promoter.

The loxP sequence elements included in the targeting vectors enable Cre-mediated deletion of the mouse CYP2C cluster, to allow the expression of CYP2C9 in the absence of the corresponding mouse genes (Figure 8).

Subsequently, the FRT sequence elements enable Flp-mediated deletion of the human expression cassette, resulting in a complete knockout at the mouse CYP2C locus (Figure 8).

### Example 3: CYP2D6 humanisation using the corresponding human promoter and knockout of the mouse CYP2D cluster

The mouse CYP2D cluster was flanked with loxP and FRT sites and a CYP2D6 expression cassette was inserted at one end of the mouse cluster, as shown in Figure 9, enabling expression of human CYP2D6 under the control of the 9kb human CYP2D6 promoter.

The loxP sequence elements included in the targeting vectors enable Cre-mediated deletion of the mouse Cyp2d cluster, to allow the expression of CYP2D6 in the absence of the corresponding mouse genes. Subsequently, the FRT sequence elements enable Flp-mediated deletion of the human expression cassette, resulting in a complete knockout at the mouse CYP2D locus.

Use of specific primers has allowed analysis of the mouse chromosome by PCR. The results demonstrate successful introduction of the desired human replacement CYP2D gene sequence and the deletion of the mouse Cyp2d Cluster (see Figures 10A and 10B). Figure 10C demonstrates that the human CYP2D6 protein is successfully expressed in the liver of these mice.

### Example 4: CYP3A4 humanisation using the mouse Cyp3a11 promoter and knockout of the mouse CYP3A cluster

A DNA sequence encoding human CYP3A4 is inserted into the mouse Cyp3a cluster at the Cyp3a11 locus, as shown in Figure 10, enabling expression of human CYP3A4 under the control of the mouse Cyp3a11 promoter. This targeting strategy is designed to allow the expression of CYP3A4 in male mice, which may not be possible using the CYP3A4 promoter.

The DNA sequence encoding human CYP3A4 comprises at least part of intron 1 of the human CYP3A4 gene (see Figure 10).

Due to the inclusion of FRT sites in the targeting vector as shown in Figure 10, the selection marker (neo^{R} in Figure 10) can be deleted by crossing with an FLP deleter strain, subsequent to insertion of human CYP3A4 into the mouse Cyp3a11 locus of the wild type.

Due to the inclusion of both loxP and FRT sites in the targeting vector as shown in the Figure, most of mouse Cyp3a11 cluster can be deleted by crossing with an FLP deleter strain or a Cre deleter strain, if the human CYP3A4 is inserted into Cyp3a cluster 5' targeted cells.

## Claims

1. A method of humanising a mouse for a gene of interest, comprising:
a) incorporating a pair of site-specific recombination sites into the mouse genome by homologous recombination such that the mouse target gene sequence that is to be replaced is flanked on each side by a recombination site;
wherein at least one of the two recombination sites is constructed so as to be contiguous with a human replacement gene sequence;
and said human replacement gene sequence is positioned so that said recombination site lies between said human replacement gene sequence and said mouse target gene sequence;
b) effecting recombination between the site-specific recombination sites such that the human sequence is introduced at the position in the mouse genome previously occupied by the mouse target gene, flanked by the residual site-specific recombination site.

2. The method of claim 1, wherein the human replacement gene sequence is inserted at the point in the mouse chromosome where the endogenous equivalent mouse target gene naturally occurs.

3. The method of claim 1 or 2, wherein the human replacement gene sequence contains a cluster of genes, optionally a cluster of genes encoding proteins involved in drug metabolism such as a cluster of genes which is part of a cytochrome P450 (*CYP*) gene cluster.

4. The method of any one of the preceding claims wherein transcription of said human replacement gene sequence is under the control of one or more endogenous mouse regulatory sequence(s) or the endogenous human regulatory sequence(s).

5. The method of any one of the preceding claims, wherein each site-specific recombination site is constructed so as to be contiguous with a selectable marker.

6. The method of claim 5 wherein each selectable marker is positioned so that said selectable marker lies between said mouse target sequence and said recombination site.

7. The method of claim 5 or claim 6, wherein each of said selectable markers are different from each other.

8. The method of claim 1 wherein a second pair of site-specific recombination sites are incorporated into the mouse genome; wherein each of the second pair of recombination sites flank the first pair of recombination sites such that the human replacement sequence may be excised by effecting site-specific recombination between this second pair of sites.

9. The method of claim 8 wherein the human replacement gene sequence is positioned between one half of the first pair of site-specific recombination sites and one half of the second pair of site-specific recombination sites.

10. The method of claim 8 or claim 9, further comprising effecting a second round of recombination between the second pair of site-specific recombination sites such that the human replacement gene sequence is excised and the gene of interest is knocked out.

11. The method of any one of the preceding claims wherein the site-specific recombination event takes place in a mouse embryonic stem cell.

12. The method of claim 11, wherein the embryonic stem cell is subsequently inserted into a blastocyst, and optionally wherein the blastocyst is subsequently transplanted into a pseudo-pregnant mouse.

13. The method of any one of the preceding claims, wherein the site-specific recombination event is effected *in vivo* and the site-specific recombination event is effected by crossing a transgenic mouse generated according to any one of the preceding claims with a mouse expressing a site-specific recombinase that recognises the site-specific recombination sites used when incorporating the human replacement gene sequence into the genome of the transgenic mouse.

14. The method of claim 13, wherein expression of the site-specific recombinase is limited to a particular tissue.

15. The method of claim 14, wherein the recombinase is albumin-Cre.

## Patentansprüche

1. Verfahren zur Humanisierung einer Maus für ein interessierendes Gen, wobei man:
a) ein Paar stellenspezifische Rekombinationsstellen mittels homologer Rekombination in das Mausgenom so einbaut, dass die auszutauschende Maus-Zielgensequenz auf jeder Seite von einer Rekombinationsstelle flankiert wird;
wobei wenigstens eine der beiden Rekombinationsstellen so konstruiert ist, dass sie an eine menschliche Austauschgensequenz grenzt; und die menschliche Austauschgensequenz so positioniert ist, dass die Rekombinationsstelle zwischen der menschlichen Austauschgensequenz und der Maus-Zielgensequenz liegt;
b) die Rekombination zwischen den stellenspezifischen Rekombinationsstellen durchführt, so dass die menschliche Sequenz an der zuvor von dem Maus-Zielgen besetzten Position im Mausgenom eingeführt wird, flankiert von der noch vorhandenen stellenspezifischen Rekombinationsstelle.

2. Verfahren nach Anspruch 1, wobei die menschliche Austauschgensequenz an dem Punkt im Mauschromosom, wo das endogene äquivalente Maus-Zielgen natürlicherweise vorkommt, eingesetzt wird.

3. Verfahren nach Anspruch 1 oder 2, wobei die menschliche Austauschgensequenz einen Cluster von Genen, gegebenenfalls einen Cluster von an Arzneistoffmetabolismus beteiligte Proteine codierenden Genen, wie etwa einen Cluster von Genen, der Teil eines Cytochrom-P450-(CYP-)Gen-Clusters ist, enthält.

4. Verfahren nach einem der vorhergehenden Ansprüche, wobei die Transkription der menschlichen Austauschgensequenz unter der Kontrolle einer endogenen oder mehrerer endogener Maus-Regulations-sequenz(en) oder der (den) endogenen menschlichen Regulationssequenz(en) steht.

5. Verfahren nach einem der vorhergehenden Ansprüche, wobei jede stellenspezifische Rekombinationsstelle so konstruiert ist, dass sie an einen selektionierbaren Marker grenzt.

6. Verfahren nach Anspruch 5, wobei jeder selektionierbare Marker so positioniert ist, dass der selektionierbare Marker zwischen der Maus-Zielsequenz und der Rekombinationsstelle liegt.

7. Verfahren nach Anspruch 5 oder Anspruch 6, wobei sich die selektionierbaren Marker jeweils voneinander unterscheiden.

8. Verfahren nach Anspruch 1, wobei ein zweites Paar stellenspezifische Rekombinationsstellen in das Mausgenom eingebaut wird; wobei das zweite Paar Rekombinationsstellen jeweils das erste Paar Rekombinationsstellen so flankiert, dass die menschliche Austauschsequenz mittels Durchführen einer stellenspezifischen Rekombination zwischen diesem zweiten Paar Stellen ausgeschnitten werden kann.

9. Verfahren nach Anspruch 8, wobei die menschliche Austauschgensequenz zwischen einer Hälfte des ersten Paars stellenspezifischer Rekombinationsstellen und einer Hälfte des zweiten Paars stellenspezifischer Rekombinationsstellen positioniert ist.

10. Verfahren nach Anspruch 8 oder Anspruch 9, bei dem man ferner eine zweite Rekombinationsrunde zwischen dem zweiten Paar stellenspezifischer Rekombinationsstellen durchführt, so dass die menschliche Austauschgensequenz ausgeschnitten und das interessierende Gen ausgeschaltet wird.

11. Verfahren nach einem der vorhergehenden Ansprüche, wobei das stellenspezifische Rekombinationsereignis in einer embryonalen Maus-Stammzelle stattfindet.

12. Verfahren nach Anspruch 11, wobei die embryonale Stammzelle anschließend in eine Blastozyste eingesetzt wird und wobei gegebenenfalls die Blastozyste anschließend in eine scheinträchtige Maus transplantiert wird.

13. Verfahren nach einem der vorhergehenden Ansprüche, wobei das stellenspezifische Rekombinationsereignis in vivo durchgeführt wird, und zwar indem man eine gemäß einem der vorhergehenden Ansprüche erzeugte transgene Maus mit einer eine stellenspezifische Rekombinase, die die beim Einbau der menschlichen Austauschgensequenz in das Genom der transgenen Maus verwendeten stellenspezifischen Rekombinationsstellen erkennt, exprimierenden Maus kreuzt.

14. Verfahren nach Anspruch 13, wobei die Expression der stellenspezifischen Rekombinase auf ein bestimmtes Gewebe beschränkt ist.

15. Verfahren nach Anspruch 14, wobei es sich bei der Rekombinase um Albumin-Cre handelt.

## Revendications

1. Procédé pour humaniser une souris pour un gène d'intérêt, comprenant :
a) l'incorporation d'une paire de sites de recombinaison spécifique de site dans le génome d'une souris, par recombinaison homologue, de telle sorte que la séquence du gène cible de la souris qui doit être remplacé soit flanquée, sur chaque côté, par un site de recombinaison ;
au moins l'un des deux sites de recombinaison étant construit de façon à être contigu d'une séquence d'un gène de remplacement humain ;
et ladite séquence d'un gène de remplacement humain étant positionnée de telle sorte que ledit site de recombinaison se trouve entre ladite séquence d'un gène de remplacement humain et ladite séquence d'un gène cible de souris ;
b) la mise en oeuvre d'une recombinaison entre les sites de recombinaison spécifique de site, de telle sorte que la séquence humaine soit introduite sur la position du génome de souris qui précédemment était occupée par le gène cible de souris, flanquée du site de recombinaison résiduel spécifique de site.

2. Procédé de la revendication 1, dans lequel la séquence du gène de remplacement humain est insérée en le point du chromosome de la souris où apparaît naturellement le gène cible de souris endogène équivalent.

3. Procédé de la revendication 1 ou 2, dans lequel la séquence du gène de remplacement humain contient un amas de gènes, en option un amas de gènes codant pour les protéines impliquées dans le métabolisme médicamenteux, tel qu'un amas de gènes faisant partie d'un amas de gènes du cytochrome P450 (CYP).

4. Procédé de l'une quelconque des revendications précédentes, dans lequel la transcription de ladite séquence du gène de remplacement humain se fait sous le contrôle d'une ou plusieurs séquences régulatrices endogènes de souris, ou d'une ou plusieurs séquences régulatrices humaines endogènes.

5. Procédé de l'une quelconque des revendications précédentes, dans lequel chaque site de recombinaison spécifique de site est construit de façon à être contigu d'un marqueur sélectionnable.

6. Procédé de la revendication 5, dans lequel chaque marqueur sélectionnable est positionné de façon que ledit marqueur sélectionnable se trouve entre ladite séquence cible de souris et ledit site de recombinaison.

7. Procédé de la revendication 5 ou 6, dans lequel lesdits marqueurs sélectionnables sont différents l'un de l'autre.

8. Procédé de la revendication 1, dans lequel une deuxième paire de sites de recombinaison spécifique de site sont incorporés dans le génome de souris, chacun des sites de la deuxième paire de sites de recombinaison flanquant la première paire de sites de recombinaison de telle sorte que la séquence de remplacement humaine puisse être excisée par mise en oeuvre d'une recombinaison spécifique de site entre les sites de cette deuxième paire de sites.

9. Procédé de la revendication 8, dans lequel la séquence du gène de remplacement humain est positionnée entre une moitié de la première paire de sites de recombinaison spécifique de site et une moitié de la deuxième paire de sites de recombinaison spécifique de site.

10. Procédé de la revendication 8 ou 9, qui comprend en outre la mise en oeuvre d'une deuxième opération de recombinaison entre la deuxième paire de sites de recombinaison spécifique de site de telle sorte que la séquence du gène de remplacement humain soit excisée et que le gène d'intérêt soit invalidé.

11. Procédé de l'une quelconque des revendications précédentes, dans lequel l'événement de recombinaison spécifique de site a lieu dans une cellule souche embryonnaire de souris.

12. Procédé de la revendication 11, dans lequel la cellule souche embryonnaire est ultérieurement insérée dans un blastocyste, et en option dans lequel le blastocyste est ultérieurement transplanté dans une souris pseudo-gravide.

13. Procédé de l'une quelconque des revendications précédentes, dans lequel l'événement de recombinaison spécifique de site est mis en oeuvre *in vivo*, et l'événement de recombinaison spécifique de site est mis en oeuvre par croisement d'une souris transgénique, produite selon l'une quelconque des revendications précédentes, avec une souris exprimant une recombinase spécifique de site qui reconnaît les sites de recombinaison spécifique de site utilisés lors de l'incorporation de la séquence du gène de remplacement humain dans le génome de la souris transgénique.

14. Procédé de la revendication 13, dans lequel l'expression de la recombinase spécifique de site est limitée à un tissu particulier.

15. Procédé de la revendication 14, dans lequel la recombinase est l'albumine-Cre.
